# NOUVEAU FASCICULE DE BREVET EUROPEEN

(11) **EP 3 806 891 B2**
(45) Date de publication et mention de la décision concernant l'opposition: **04.03.2026**
(45) Mention de la délivrance du brevet: 02.08.2023
(21) Numéro de dépôt: 19742443.5
(22) Date de dépôt: 14.06.2019
(51) Int. Cl.: A61K 38/46, C07K 16/36, A61K 39/00, A61P 7/04, A61K 39/395

(54) **COMBINAISON DE FACTEUR VII ET D'UN ANTICORPS BISPÉCIFIQUE ANTI-FACTEURS IX ET X**
KOMBINATION VON BLUTGERINNUNGSFAKTOR VII MIT EINEM BISPEZIFISCHEN ANTIKÖRPER GEGEN BLUTGERINNUNGSFAKTOR IX UND X
COMBINATION OF FACTOR VII AND A ANTI-FACTOR IX AND FACTOR X BISPECIFIC ANTIBODY

(30) Priorité: 14.06.2018 FR 1855239
(43) Date de publication de la demande: 21.04.2021
(73) Titulaire: Laboratoire Français du Fractionnement et des Biotechnologies, 92800 Puteaux (FR)
(72) Inventeur: PLANTIER, Jean-Luc, 59170 CROIX (FR)
(74) Mandataire: Cabinet Becker et Associés
(86) Numéro de dépôt international: PCT/FR2019/051456
(87) Numéro de publication internationale: WO 2019/239080

(56) Documents cités:
- EP-A1- 2 687 595
- JOHANNES OLDENBURG, MAHLANGU JOHNNY N., KIM BENJAMIN, SCHMITT CHRISTOPHE, CALLAGHAN MICHAEL U., YOUNG GUY, SANTAGOSTINO ELENA, KRU: "Emicizumab Prophylaxis in Hemophilia A with Inhibitors", THE NEW ENGLAND JOURNAL OF MEDICINE, MASSACHUSETTS MEDICAL SOCIETY, US, vol. 377, no. 9, 31 August 2017 (2017-08-31), US , pages 809 - 818, XP055686025, ISSN: 0028-4793, DOI: 10.1056/NEJMoa1703068
- HARTMAN R., S. KNAPPE, B. GOLDSTEIN, B. M. EWENSTEIN, L. VALENTINO, F. SCHEIFLINGER: "OR36 Synergistic Effects of a Procoagulant Bispecific Antibody and FEIBA or Factor VIIA on Thrombin Generation", HAEMOPHILIA, BLACKWELL SCIENCE, OXFORD, GB, vol. 23, no. S2, 1 February 2017 (2017-02-01) - 3 February 2017 (2017-02-03), GB , pages 26 - 26, XP093275550, ISSN: 1351-8216, DOI: 10.1111/hae.13158
- HARTMANN R ET AL: "In vitro studies show synergistic effects of a procoagulant bispecific antibody and bypassing agents.", JOURNAL OF THROMBOSIS AND HAEMOSTASIS : JTH 11 JUN 2018, 11 June 2018 (2018-06-11), XP002788933, ISSN: 1538-7836
- DATABASE BIOSIS [online] BIOSCIENCES INFORMATION SERVICE, PHILADELPHIA, PA, US; 7 December 2017 (2017-12-07), SANTAGOSTINO ELENA ET AL: "Management of Joint Replacement in Hemophilia a with Inhibitors during Emicizumab Prophylaxis", XP002788934, Database accession no. PREV201900188250
- LANGER A L [0000-0002-0301-7073] ET AL: "Evaluating the safety of emicizumab in patients with hemophilia A", EXPERT OPINION ON DRUG SAFETY 20181202 TAYLOR AND FRANCIS LTD GBR, vol. 17, no. 12, 2 December 2018 (2018-12-02), pages 1233 - 1237, XP002788935, ISSN: 1474-0338

## Description

L'invention concerne des compositions pharmaceutiques utiles pour le traitement d'un désordre de la coagulation, comme l'hémophilie A, spécialement chez un patient présentant une hémophilie de type A avec développement d'anticorps inhibiteurs du facteur VIII. L'invention est telle que définie dans les revendications.

### Arrière-plan technologique

La coagulation met en jeu deux voies, l'une intrinsèque, l'autre extrinsèque, aboutissant à une voie finale commune. La combinaison des deux mécanismes assure la formation d'un caillot de sang solide et flexible, qui résiste à la pression sanguine. Sous l'action de la thrombine, le fibrinogène subit des modifications chimiques qui aboutissent à la formation de la fibrine. La fibrine est nécessaire à la formation du caillot.

La voie intrinsèque comporte les facteurs présents dans la circulation et le processus de coagulation démarre au sein même du vaisseau sanguin. La voie extrinsèque quant à elle, met en jeu les facteurs tissulaires non présents normalement dans la circulation et qui sont libérés lors d'une lésion vasculaire.

Le facteur VII est une glycoprotéine qui intervient dans la voie extrinsèque de la coagulation sanguine. Pour initier la cascade de coagulation, le FVII doit être activé en FVIIa. Une fois activé, le FVIIa se complexe au facteur tissulaire (FT), protéine associée à des phospholipides, qui est libérée lors de la lésion vasculaire. Le FVlla seul (non complexé au facteur tissulaire) présente une faible activité protéolytique. Le complexe FVIIa-FT transforme ensuite le facteur X en facteur Xa en présence d'ions calcium. Ce complexe agit également sur l'activation du facteur FIX en FIXa, catalysant ainsi la voie intrinsèque. Les facteurs IXa et Xa, activent en retour le facteur VII activé.

Le facteur IX et le facteur X interviennent dans la voie intrinsèque de la coagulation. Le facteur IX activé permet l'activation du facteur X en facteur Xa.

Le facteur Xa complexé au facteur FV activé et à la prothrombinase transforme la prothrombine en thrombine. La thrombine agit alors sur le fibrinogène pour le transformer en fibrine et permet également l'activation du FVIII et du FV en FVllla et FVa, respectivement. La thrombine, pour sa part, permet d'activer le facteur XIII en FXllla, responsable de la consolidation du caillot de fibrine, en présence de calcium, naturellement présent dans le plasma.

Néanmoins, lorsqu'un facteur de la coagulation fait défaut, la cascade de coagulation est interrompue ou déficiente et on parle alors d'une coagulation anormale.

Le facteur VII activé agit localement en présence de facteur tissulaire libéré après une lésion de tissus engendrant des hémorragies, même en l'absence de facteur VIII ou IX. C'est pourquoi le facteur VII, de préférence sous forme activée, est utilisé pour le traitement de certains troubles de la coagulation sanguine se manifestant par des saignements.

Le facteur VII est ainsi utilisé dans le traitement des patients atteints d'hémophilie, présentant un déficit en facteur VIII (hémophilie de type A) ou en facteur IX (hémophilie de type B), ainsi que des patients présentant d'autres déficiences des facteurs de coagulation, par exemple un déficit héréditaire en FVII. Le FVII est également préconisé dans le traitement d'accidents vasculaires cérébraux.

Certains patients hémophiles développent des anticorps inhibiteurs vis-à-vis du Facteur VIII administré, généralement sous forme concentrée, comme traitement de l'hémophilie. C'est aujourd'hui la complication la plus fréquente du traitement de l'hémophilie.

Des anticorps bispécifiques ciblant le FIX ou le FIXa et le FX ou le FXa, tels que l'Emicizumab, sont utilisés pour traiter des patients atteints d'hémophilie A, présentant des inhibiteurs anti-facteur VIII. Ces anticorps se substituent fonctionnellement au FVIII en favorisant l'activation du FX par le FIXa par rapprochement de ces deux molécules. Ces anticorps ont une action de longue durée.

La combinaison FVIIa recombinant issu de culture cellulaire (tel que Novoseven^{®}, produit dans des cellules BHK) avec de l'Emicizumab (tel que ACE910 ou Hemlibra^{®}) a été testée *(*R. HARTMANN, et al. OR36 / Synergistic Effects of a Procoagulant Bispecific Antibody and FEIBA or Factor VIIA on Thrombin Generation (Haemophilia (2017), 23 (Suppl. 2), 11-27*))*. Cette combinaison ne montre qu'un effet additif sur le traitement des troubles de la coagulation.

Il existe par conséquent le besoin d'une combinaison pharmaceutique permettant une meilleure prise en charge des patients hémophiles A, et plus particulièrement des patients présentant des anti-facteur VIII.

### Résumé de l'invention

L'invention propose de combiner du facteur VII transgénique avec un anticorps multispecifique dirigé contre les facteurs IX et X, ledit facteur VII transgénique étant un facteur VII humain issu d'une production par les cellules épithéliales des glandes mammaires d'un lapin transgénique.

Selon l'invention, la combinaison de l'invention de facteur VII obtenu par transgenèse et d'anticorps dirigé contre le facteur IX et le facteur X induit un effet synergique dans le traitement des troubles de la coagulation, et particulièrement pour le traitement des patients atteints d'hémophilie A présentant des inhibiteurs anti-FVIII et de patients déficitaires en FVII.

Un aspect de l'invention est donc une composition pharmaceutique comprenant :
a. du facteur VII transgénique, ledit facteur VII transgénique étant un facteur VII humain issu d'une production par les cellules épithéliales des glandes mammaires d'un lapin transgénique, et
b. un anticorps multispécifique, de préférence bispécifique, dirigé contre le facteur IX et le facteur X, tel que par exemple l'emicizumab.

De préférence, le facteur VII est sous la forme de facteur VII activé (FVlla).

Dans un mode de réalisation particulier, le facteur IX est sous la forme de facteur IX activé (FIXa), et/ou le facteur X est sous la forme de facteur X activé (FXa).

Ledit facteur VII transgénique est un facteur VII humain issu d'une production par les cellules épithéliales des glandes mammaires d'un un lapin transgénique pour le facteur VII humain.

L'invention fournit également un produit de combinaison comprenant :
a. du facteur VII transgénique, ledit facteur VII transgénique étant un facteur VII humain issu d'une production par les cellules épithéliales des glandes mammaires d'un lapin transgénique, et
b. un anticorps multispécifique dirigé contre le facteur IX et le facteur X,
pour son utilisation dans la prévention ou le traitement d'un désordre de la coagulation, telle que l'hémophilie A, plus spécialement l'hémophilie A avec inhibiteurs de facteur VIII (FVIII).

De préférence, le produit de combinaison est sous la forme d'une composition pharmaceutique qui comprend à la fois le facteur VII transgénique et ledit anticorps.

De manière alternative, le facteur VII transgénique et l'anticorps sont sous la forme de compositions séparées, adaptées à une administration simultanée ou séparée (par exemple séquentielle) au patient.

### Liste des Figures :

Un autre objet de l'invention vise encore un kit comprenant :
- Un récipient contenant un facteur FVII transgénique, ledit facteur VII transgénique étant un facteur VII humain issu d'une production par les cellules épithéliales des glandes mammaires d'un lapin transgénique; et
- Un autre récipient contenant un anticorps dirigé contre le facteur IX et le facteur X.

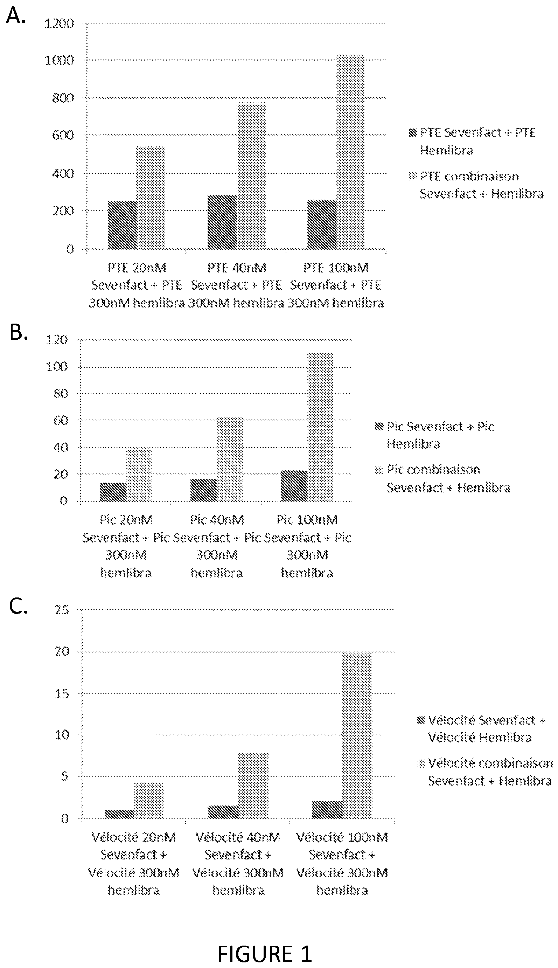

Figure 1 : Evaluation de l'effet thrombogène synergique de la combinaison Sevenfact^{™} + Hemlibra^{®} sur le lot 1 de plasma Hémophile A après induction au TF/PL. (A) Evaluation de l'effet thrombogène synergique sur le PTE, (B) Evaluation de l'effet thrombogène synergique sur le pic de génération de thrombine, (C) Evaluation de l'effet thrombogène synergique sur la vélocité.

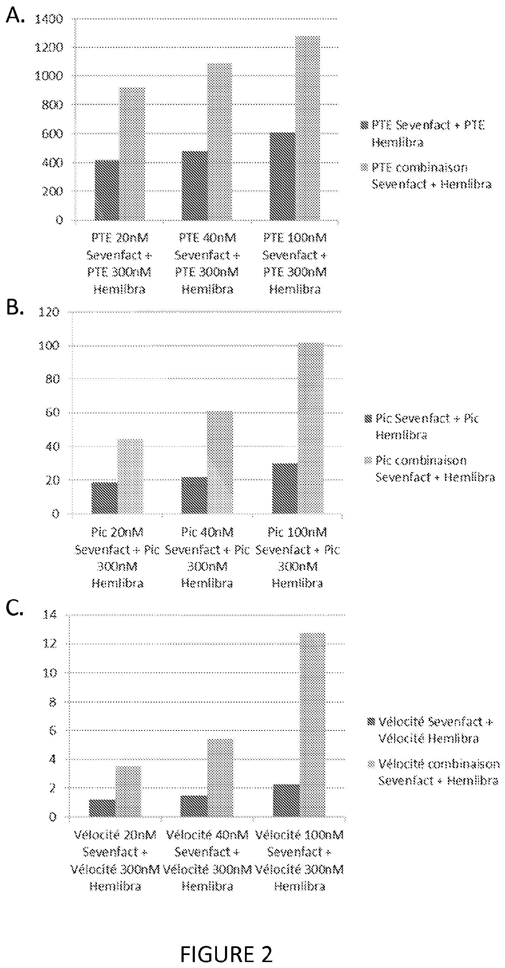

Figure 2 : Evaluation de l'effet thrombogène synergique de la combinaison Sevenfact^{™} + Hemlibra^{®} sur le lot 2 de plasma Hémophile A après induction au TF/PL. (A) Evaluation de l'effet thrombogène synergique sur le PTE, (B) Evaluation de l'effet thrombogène synergique sur le pic de génération de thrombine, (C) Evaluation de l'effet thrombogène synergique sur la vélocité.

### Description détaillée de l'invention

### Définitions générales

Le phénomène de coagulation comporte une cascade de réactions enzymatiques impliquant des facteurs de la coagulation présents sous forme de proenzymes qui, en présence de certains cofacteurs, sont convertis par clivage protéolytique en leur forme « **activée** ». La forme activée de chaque facteur présent sous la forme de précurseur inactif est désignée par la lettre a. Ainsi le FVlla résulte, in vivo, du clivage du zymogène par différentes protéases (FIXa, FXa, FVlla) en deux chaînes réunies par un pont disulfure.

Le terme " **traitement** " ou " **traiter** " désigne généralement une amélioration, la prophylaxie, ou l'inversion d'une maladie ou d'un trouble, ou au moins d'un symptôme, par exemple le ralentissement de la progression d'une maladie, ou la stabilisation d'un symptôme. Est également inclus le retard de l'apparition d'une maladie ou trouble, ou d'au moins un symptôme.

Le terme **"prévention"** ou " **prévenir** " désigne une réduction du risque de développement ou d'acquisition d'une maladie ou d'un trouble spécifié.

Au sens de la présente invention, par « **patient** » ou « **sujet** », on entend tout mammifère, et plus particulièrement les êtres humains, hommes ou femmes, de tout âge, y compris les enfants.

Le terme « **composition pharmaceutique** » fait référence aux préparations permettant l'activité biologique des ingrédients actifs et ne contenant aucun composant additionnel toxique pour les sujets auxquels la composition est administrée.

### Facteur VII transgénique

Le terme **"Facteur VII "** ou **"FVII"** inclut les polypeptides comprenant la séquence 1-406 du facteur VII humain de type sauvage (comme décrit dans le brevet U. S. 4,784, 950), ou du FVII dérivé d'une autre espèce (par exemple bovin, porcin, canin, murin). Il comprend en outre les variations alléliques naturelles du facteur VII qui peuvent exister, et toute forme ou degré de glycosylation ou autre modification post-traductionnelle. Le terme « Facteur VII » inclut aussi les variants du FVII qui présente la même ou une activité biologique supérieure par rapport à l'activité de la forme sauvage, ces variants incluant notamment les polypeptides différant du FVII sauvage par insertion, délétion, ou substitution d'un ou plusieurs acides aminés.

Sauf indication contraire, dans la présente description, le terme « Facteur VII » se réfère indifféremment au FVII non clivé (zymogène) ou au Facteur VII activé (FVlla).

Le FVlla est donc composé d'une chaîne légère de 152 acides aminés de poids moléculaire d'environ 20 kDa et d'une chaîne lourde de 254 acides aminés de poids moléculaire d'environ 30 kDa liées entre elles par un seul pont disulfure (Cys135-Cys262).

Par « **facteur VII recombinant** », il est entendu tout facteur VII issu du génie génétique et résultant de l'expression du gène correspondant dans tout microorganisme, plante ou plante transgénique. Par microorganisme, il est entendu tout système bactérien, fongique, viral ou cellulaire. Le facteur VII recombinant peut également être produit à partir de cellules eucaryotes en culture telles que des cellules de plante ou de mammifère, par exemple des cellules animales ou humaines.

Par « **facteur VII transgénique** », il est entendu tout facteur VII recombinant obtenu à partir d'un animal transgénique pour le facteur VII.

Par « **animal transgénique** », on entend tout animal non-humain présentant une modification de son génome destinée à permettre l'expression d'une protéine d'intérêt (ici du facteur VII). La modification du génome peut résulter d'une altération, d'une modification ou de l'insertion d'un gène. Cette modification peut être due à l'action d'agents altérants ou mutagènes classiques ou bien effectuée par mutagenèse dirigée. La modification du génome peut également résulter d'une insertion de gène(s) ou de remplacement de gène(s) dans sa (leur) forme sauvage ou mutée. Selon l'invention, l'animal transgénique est le lapin.

Par l'expression « **activité biologique du facteur VIIa** », il est entendu la capacité du FVlla à générer de la thrombine, par exemple à la surface des plaquettes activées. L'activité du facteur VII peut être évaluée de différentes manières. L'activité biologique du FVlla peut par exemple être quantifiée en mesurant la capacité d'une composition de FVII à promouvoir la coagulation du sang en utilisant un plasma déficient en FVII et en thromboplastine, comme décrit par exemple dans le brevet US No. 5,997,864. Dans cet essai, l'activité biologique est évaluée par rapport à un échantillon témoin et est converti en « unités de FVII » par comparaison avec un sérum humain standard poolé contenant 1 unité/ml d'activité de Facteur VII. Alternativement, l'activité biologique du facteur VII peut être quantifiée par (i) la mesure de la capacité du Facteur VIIa à produire du facteur Xa dans un système comprenant du facteur tissulaire (FT) englobé dans une membrane lipidique et du Facteur X (Persson et al. J. Biol. Chem. 272 : 19919- 19924, 1997) ; (ii) en mesurant l'hydrolyse du facteur X dans un système aqueux ; (iii) en mesurant la liaison physique du FVlla au FT par l'intermédiaire de la résonance plasmon de surface (Persson , FEBS letts, 413 :359-363, 1997), (iv) en mesurant l'hydrolyse d'un substrat synthétique ou (v) en mesurant la génération de thrombine dans un système in vitro indépendant du FT.

Dans un mode de réalisation préféré, le FVII décrit ici est un polypeptide dont la séquence peptidique peut être celle du FVII humain naturel, c'est-à-dire la séquence présente chez les humains ne présentant pas de troubles liés au FVII. Une telle séquence est décrite dans le document EP 0 200 421.

Avantageusement, la séquence du FVII utilisée dans l'invention est la séquence SEQ ID NO : 1.

Par « **synergie** », ou « **effet synergique** » il est de préférence entendu un effet de la combinaison de deux produits supérieur, à 2 fois la somme des effets de chacun des produits pris isolément. Selon la présente invention, un effet synergique est obtenu lorsque l'utilisation d'un FVlla transgénique en combinaison avec un anticorps multispécifique dirigé contre le facteur IX et le facteur X permet d'obtenir un effet supérieur à 2 fois la somme de l'effet obtenu avec un FVlla transgénique seul et l'effet obtenu avec un anticorps multispécifique dirigé contre le facteur IX et le facteur X seul, sur au moins un paramètre de génération de thrombine. Le paramètre de génération de thrombine est choisi parmi la hauteur de pic, la vélocité, ou le potentiel thrombine endogène (PTE).

Dans un mode de réalisation particulier, le FVlla est administré à une concentration inférieure ou égale à 105 nM, de préférence inférieure à 100 nM.

Dans un mode de réalisation particulier, l'anticorps multipécifique dirigé contre le facteur IX et le facteur X est administré à une concentration inférieure à 600 nM, de préférence inférieure à 550 nM, de préférence inférieure à 500 nM, de préférence inférieure à 450 nM, de préférence inférieure à 400 nM, de préférence inférieure à 350 nM, de préférence inférieure à 325nM.

Selon l'invention, le facteur VII est obtenu à partir du lait d'un lapin transgénique.

Une méthode de production d'une protéine recombinante dans le lait d'un animal transgénique peut comporter les étapes suivantes : une molécule d'ADN synthétique comprenant un gène codant pour une protéine d'intérêt (ici par exemple le FVII humain), ce gène étant sous le contrôle d'un promoteur d'une protéine sécrétée naturellement dans le lait, est intégrée dans un embryon d'un mammifère non humain. L'embryon est ensuite placé dans une femelle de mammifère de la même espèce. Une fois que le mammifère issu de l'embryon s'est suffisamment développé, la lactation du mammifère est induite, puis le lait recueilli. Le lait contient alors le FVII d'intérêt sécrété par l'animal transgénique.

Un exemple de préparation de protéine dans le lait d'une femelle de mammifère autre que l'être humain est donné dans la demande de brevet EP0527063, dont l'enseignement peut être repris pour la production du facteur VII de l'invention.

La sécrétion de facteur VII par les glandes mammaires, permettant sa sécrétion dans le lait du mammifère transgénique, implique le contrôle de l'expression du facteur VII de manière tissu-dépendante. De telles méthodes de contrôle sont bien connues de l'homme du métier. Le contrôle de l'expression est effectué grâce à des séquences permettant l'expression de la protéine vers un tissu particulier. Il s'agit notamment des séquences promotrices WAP, béta-caséine, béta-lactoglobuline et des séquences peptide signal, la liste n'étant pas limitative.

Selon l'invention, le facteur VII est produit dans le lait de lapines transgéniques.

De manière particulièrement avantageuse, l'expression dans les glandes mammaires de la lapine est effectuée sous le contrôle du promoteur de la béta-caséine bien connu de l'homme du métier. En particulier, un plasmide contenant le promoteur béta-caséine est fabriqué par introduction d'une séquence comportant le promoteur du gène béta-caséine, ce plasmide étant réalisé de manière à pouvoir recevoir un gène étranger placé sous la dépendance de ce promoteur. Le gène codant pour le FVII humain est intégré, et placé sous la dépendance du promoteur béta-caséine. Le plasmide contenant le promoteur et la séquence codant pour la protéine d'intérêt est digéré par des enzymes de restriction pour libérer le fragment d'ADN contenant le promoteur béta-caséine et la séquence de FVII humain. Après purification, les fragments sont introduits, par microinjection dans le pronucléus mâle d'embryons de lapines Wild-type. Les embryons sont ensuite cultivés avant transfert dans l'oviducte de femelles Wild-type hormonalement préparées. A la mise bas de ces femelles, la descendance est évaluée par PCR pour déterminer les animaux transgéniques. Le nombre de copies du transgène et son intégrité est révélée par la technique de Southern à partir de l'ADN extrait des lapereaux transgéniques obtenus. La concentration de FVII humain exprimé dans le lait des descendants femelles transgéniques est évaluée à l'aide de tests immuno-enzymatiques.

Dans un mode de réalisation particulier, le facteur VII utile dans l'invention est obtenu par un procédé comprenant les étapes suivantes :
(a) l'insertion d'une séquence d'ADN comprenant un gène codant pour le facteur VII dans un embryon de mammifère non-humain, ledit gène étant sous le contrôle transcriptionnel du promoteur béta-caséine,
(b) le transfert d'embryons obtenus à l'étape a) dans l'oviducte de femelles de mammifère non humain de manière à ce qu'il se développe en un mammifère adulte non humain,
(c) l'induction de la lactation chez le mammifère adulte non humain obtenu à l'étape b) de type femelle ou dans un descendant femelle de ce mammifère non-humain dans lequel le gène et le promoteur sont présents dans son génome,
(d) la collecte du lait dudit mammifère non humain, et
(e) la purification du FVII présent dans le lait collecté.
Selon l'invention, le mammifère non humain est un lapin.

Le FVII transgénique utile ici présente un point isoélectrique substantiellement homogène.

Par « **point isoélectrique** » ou « **pl** », il est entendu le pH pour lequel la charge élémentaire nette de la molécule de facteur VII ou de facteur VIIa est nulle, c'est-à-dire le pH auquel la molécule est électriquement neutre (forme zwitterionique). Le point isoélectrique du facteur VII selon l'invention peut être mesuré en mettant en oeuvre une technique bien connue de l'homme du métier telle que la focalisation isoélectrique ou iso-electric focusing (« IEF »). Cette technique électrophorétique permet la séparation des protéines sur la base de leur point isoélectrique. Elle consiste en une migration, induite par un courant électrique uniforme, des protéines dans un gradient de pH jusqu'à ce qu'elles atteignent un pH équivalent à leur point isoélectrique spécifique, moment auquel elles cessent de migrer puisque leur charge nette est nulle. Les gels IEF sont utilisés pour déterminer le point isoélectrique d'une protéine donnée.

Par « **substantiellement homogène** », il est entendu qu'au moins 90%, de préférence au moins 95% des molécules de facteur VII de la composition ont un point isoélectrique compris dans un écart d'unité de pH inférieure ou égale à 1,2. Dans un autre mode de réalisation de l'invention au moins 50%, de préférence au moins 55%, de préférence 60% des molécules de facteur VII transgénique de la composition ont un point isoélectrique compris dans un écart d'unité de pH inférieure à 1, de préférence inférieure à 0,5. Dans un autre mode de réalisation préféré, au moins 50%, de préférence au moins 55%, de préférence 60% des molécules de facteur VII de la composition ont un point isoélectrique compris dans un écart d'unité de pH de 0,4.

Par « **formes N-glycanniques** », il est entendu l'ensemble des formes N-glycanniques présentes sur les deux sites de N-glycosylation du facteur VII de l'invention. Les formes N-glycanniques sont dite monochargées, si leur charge totale est égale à 1. Au sens de la présente invention, par « charge », il est entendu un groupement phosphate, un groupement sulfate ou une molécule d'acide sialique. Ainsi les formes N-glycanniques sont dites monochargées, si elles contiennent uniquement un groupement phosphate ou un groupement sulfate ou une molécule d'acide sialique. Par opposition au terme « monochargée » le terme « bichargée » signifie que la charge totale portée par les formes N-glycanniques est égale à 2, c'est-à-dire qu'elles présentent deux charges choisies parmi un groupement phosphate, un groupement sulfate et/ou une molécule d'acide sialique. En d'autres termes, les formes N-glycanniques bichargées possèdent une molécule d'acide sialique et un groupement phosphate, ou une molécule d'acide sialique et un groupement sulfate, ou deux molécules d'acides sialiques, ou deux groupements phosphates, ou deux groupements sulfates, ou un groupement phosphate et un groupement sulfate. Le terme « trichargée » signifie que la charge totale portée par les formes N-glycanniques est égale à 3, c'est-à-dire qu'elles présentent trois charges choisies parmi un groupement phosphate, un groupement sulfate et/ou une molécule d'acide sialique. En d'autres termes, les formes N-glycanniques trichargées possèdent une molécule d'acide sialique et un groupement phosphate et un groupement sulfate, ou deux molécules d'acide sialique et un groupement phosphate, ou deux molécules d'acide sialique et un groupement sulfate, ou un groupement d'acide sialique et deux groupements phosphate, ou un groupement d'acide sialique et deux groupements sulfate, ou un groupement phosphate et deux groupements sulfate, ou un goupement sulfate et deux groupements phosphate ou trois molécules d'acide sialique, ou trois groupements phosphate, ou trois groupements sulfate. Le terme « neutre » quant à lui signifie que les formes N-glycanniques ne contiennent aucune charge.

La charge des formes N-glycanniques du facteur VII selon l'invention peut être mesurée en mettant en oeuvre une technique bien connue de l'homme du métier, notamment par chromatographie en phase liquide à ultra haute performance ayant une résine échangeuse d'anions couplée à une détection par fluorescence (AEX-UPLC/FD). Cette méthode permet de séparer les différentes formes N-glycanniques en fonction de leur charge apparente (voir en particulier, Hermentin et al, Glycobiology, vol.6, no. 2, 1996). Dans le cadre d'une chromatographie échangeuse d'anions, une résine chargée positivement est utilisée comme phase stationnaire. Ces résines chargées positivement sont généralement constituées d'un polymère réticulé ou gel, sur lequel des groupements chargés positivement sont greffés. Dans un mode de réalisation avantageux de l'invention, une colonne échangeuse d'anions faible de type aminopropyl est utilisée.

Dans le cas de la composition de facteur VII selon l'invention, il apparait que parmi toutes les formes N-glycanniques du facteur VII de la composition, au moins 50% des formes N-glycanniques, au moins 60% des formes N-glycanniques, de préférence au moins 65%, de préférence au moins 70%, de préférence au moins 75%, de préférence au moins 80%, de préférence au moins 85%, de préférence au moins 90%, de préférence au moins 95%, sont monochargées. Dans un mode de réalisation préféré, les molécules de facteur VII présentant des formes N-glycanniques monochargées représentent entre 50% et 95% des molécules de facteur VII de la composition, de préférence entre 50% et 90% des molécules de facteur VII de la composition, de préférence entre 50% et 80% des molécules de facteur VII de la composition, de préférence entre 50% et 75% des molécules de facteur VII de la composition, de préférence entre 50% et 70% des molécules de facteur VII de la composition, de préférence entre 50 et 65% des molécules de facteur VII de la composition, de préférence entre 50% et 60% des molécules de facteur VII de la composition.

Le point isoélectrique substantiellement homogène de la composition du facteur VII de la combinaison selon l'invention résulte de la combinaison des propriétés de glycosylation et de γ-carboxylation des molécules de FVII qui la composent.

Le facteur VII transgénique utile ici présente des caractéristiques de modifications post-traductionnelles. Il s'agit en particulier de modifications de la glycosylation, telles que deux sites de N-glycosylation avec un taux nul ou très faible de Galα1,3Gal dans la composition de FVII, ou encore assez faible pour ne pas être immunogène. Par opposition, le FVII décrit ici n'est pas un FVII plasmatique, c'est-à-dire qu'il n'est pas un produit purifié à partir de plasma humain ou animal. Plus particulièrement, le FVII transgénique utile ici présente des modifications post-traductionnelles, ainsi que deux sites de O-glycosylation à motifs glycanniques définis, une γ-carboxylation, et des ponts disulfures spécifiques.

Le FVlla utile ici peut comporter plusieurs modifications post-traductionnelles : les neuf ou dix premiers acides glutamiques N-terminaux sont γ-carboxylés, l'Asp₆₃ est partiellement hydroxylé, la Ser₅₂ et la Ser₆₀ sont O-glycosylées et portent respectivement les motifs Glucose(Xylose)₀₋₂ et Fucose, l'Asn₁₄₅ et l'Asn₃₂₂ sont N-glycosylées majoritairement par des structures complexes biantennées monosialylées.

Avantageusement, les molécules de facteur VII transgénique utiles ici présentent, pour au moins 80% d'entre elles, une γ-carboxylation sur neuf résidus d'acide glutamique. Dans un autre mode de réalisation, au moins 85% desdites molécules présentent une γ-carboxylation sur neuf résidus d'acide glutamique. Dans un autre mode de réalisation, entre 85% et 100%, de préférence entre 90% et 100%, de préférence entre 95% et 100% desdites molécules présentent une γ-carboxylation sur neuf résidus d'acide glutamique. Avantageusement, le taux de γ-carboxylation sur le résidu d'acide glutamique 35 (Glu 35) des molécules de facteur VII de la composition est inférieur à 20%. Dans un autre mode de réalisation, le taux de γ-carboxylation du résidu Glu35 est inférieur à 15%, de préférence inférieur à 10%, de préférence inférieur à 5%.

Le motif Gala1,3Gal est une structure composée de deux galactoses liés en α1,3. Il est situé à l'extrémité des antennes oligosaccharidiques des structures N-liées. Ce motif est connu pour son immunogénicité. On préfère ainsi produire un FVII ou un FVlla dont le taux de structures Galα1,3Gal est nul ou si faible qu'il ne peut être distingué du bruit de fond obtenu par les mesures mises en oeuvre par les appareillages d'analyses actuellement disponibles. Cette expression désigne de manière équivalente tout FVII transgénique dont le taux de Galα1,3Gal est proche de celui du FVII plasmatique. Avantageusement, le taux de Galα1,3Gal de la composition de FVII décrite ici n'est pas immunogène pour l'homme. De plus, le FVII utile ici comporte de préférence, comme le FVII humain, deux sites de N-glycosylation, en position 145 et 322, et 2 sites de O-glycosylation, en position 52 et 60. Dans un site de N-glycosylation, les chaînes d'oligosaccharides sont liées à une asparagine (N- liées). Dans un site de O-glycosylation, les chaînes d'oligosaccharides sont liées à une serine. Les motifs liés sur ces acides aminés seront différents pour chaque protéine de la composition. Toutefois, on peut quantifier, pour la totalité de la composition, le taux de chaque motif glycannique, ou encore de chaque sucre.

Les pourcentages des différents glycannes donnés dans la présente demande ne tiennent pas compte de la O-glycosylation.

De préférence, la composition de FVII est caractérisée en ce que, parmi tous les motifs glycanniques du FVII de la composition, au moins 40% sont des formes glycanniques biantennées, monosialylées. Dans un autre mode de réalisation, les formes biantennées monosialylées sont présentes à au moins 50%. Dans un autre mode de réalisation, les formes biantennées monosialylées sont présentes à au moins 60%, de préférence au moins 65%, de préférence au moins 70%.

Avantageusement, les formes glycanniques biantennées, monosialylées du FVII sont majoritaires. La composition de FVII se caractérise en ce qu'au moins certains des acides sialiques du facteur VII impliquent des liaisons α2-6.

Avantageusement, au moins 65% des acides sialiques du FVII impliquent des liaisons α2,6. De façon très avantageuse, au moins 70%, voire 80% et, en particulier, au moins 90% des acides sialiques du FVII impliquent des liaisons α2,6.

De façon particulièrement préférée, tous les acides sialiques impliquent des liaisons α2,6, c'est- à-dire que tous les acides sialiques sont liés au galactose par une liaison α2,6. La composition de FVII décrite ici peut en outre comprendre des acides sialiques de liaisons α2-3.

Selon des modes de réalisation de l'invention, de 65% à 100% des acides sialiques du FVII impliquent des liaisons α2,6. De façon plus préférée, de 70% ou 80% à 100% des acides sialiques du FVII impliquent des liaisons α2,6.

Avantageusement, parmi les formes glycanniques biantennées monosialylées du FVII, les formes glycanniques majoritaires sont non fucosylées.

De préférence, ces formes glycanniques biantennées monosialylées non fucosylées sont présentes dans le FVII de la composition à un taux supérieur à 20%. Avantageusement, ce taux est supérieur à 25%, ou encore supérieur à 40%. D'une manière particulièrement avantageuse, le taux de fucosylation de la composition de FVII est compris entre 20% et 50%. Dans un mode de réalisation, ce taux peut être inférieur à 20%.

Dans un mode de réalisation particulier, au moins 10%, de préférence au moins 15%, de préférence au moins 20%, de préférence au moins 25% des formes N-glycanniques des facteurs VII de la composition sont high mannose/ hybride.

De façon préférée, le profil de glycosylation décrit ici procure au FVII une activité biologique et une stabilité améliorées. Les compositions de facteur VII présentant un point isoélectrique substantiellement homogène facilitent l'étape de formulation à un pH optimal, de préférence à un pH optimal de 6.0 ± 0.2, des compositions pharmaceutiques en évitant la précipitation du FVII. En effet, il est connu qu'au point isoélectrique d'une molécule, celles-ci auront tendance à s'agréger et à précipiter. Les molécules de facteur VII utilisés dans la composition de l'invention ont un point isoélectrique compris entre 6.6 et 7.0. Il en résulte une meilleure stabilité de la composition de facteur VII, notamment lorsque celle-ci est formulée à un pH inférieur au point isoélectrique, et en particulier à pH 6.0. L'amélioration de la stabilité de la composition de facteur VII permet d'éviter les interactions électrostatiques responsables des phénomènes de précipitation et d'agrégation de type soluble et insoluble, ainsi que d'éviter la perte de matières premières et donc une baisse de rendement aboutissant à une perte de quantité en principe actif et donc potentiellement à une perte d'activité.

Le FVII transgénique est produit par la lapine dans son lait permettant d'obtenir une composition où chaque molécule de facteur VII de la composition présente deux sites de N-glycosylation. De préférence toutes les molécules de FVII de la composition ont un taux de motifs glycanniques Galα1,3Gal inférieur à 4%, voire nul. Ainsi, de manière avantageuse, le FVII transgénique produit par la lapine ne possède pas de motif glycannique Galα1,3Gal.

Le FVII peut être purifié à partir du lait par des techniques connues de l'homme du métier. Par exemple, une méthode de purification d'une protéine d'intérêt à partir de lait, telle que décrite dans le brevet US 6,268,487, peut comprendre les étapes suivantes consistant à : a) soumettre le lait à une filtration tangentielle sur une membrane de porosité suffisante pour former un retentat et un perméat, le perméat contenant la protéine exogène, b) soumettre le perméat à un appareil de capture par chromatographie de manière à déplacer la protéine exogène et obtenir un effluent, c) combiner l'effluent et le retentat, d) répéter les étapes a) à c) jusqu'à ce que le FVII soit séparé des lipides, des micelles de caséines, et que le FVII soit récupéré.

Avantageusement, le FVII de l'invention est sous forme activée. Dans un mode de réalisation, le FVII peut être activé in vitro par les facteurs Xa, Vlla, Ila, IXa ou Xlla. Le FVII peut également être typiquement activé au cours de son procédé de purification, en particulier par le passage sur des colonnes de chromatographie chargées positivement.

### Anticorps multispécifique

Par « **anticorps multispécifique** », on entend tout anticorps possédant au moins deux sites de liaison spécifique à au moins deux antigènes différents, ou épitopes différents d'un même antigène. Le terme « spécifique » signifie que l'anticorps a la capacité de reconnaitre et de lier un antigène, substantiellement sans réaction croisée avec un autre antigène. De manière avantageuse, l'anticorps présente, vis-à-vis de chaque antigène, une constante d'affinité Kd d'au moins 10⁻⁶ M, de préférence au moins 10⁻⁷ M, de préférence encore au moins 10⁻⁸ M, 10⁻⁹ M, ou 10⁻¹⁰ M.

Ainsi, les anticorps utiles dans l'invention ont la capacité de se lier spécifiquement à la fois au facteur de coagulation IX et au facteur de coagulation X sous forme activée ou non. L'anticorps utilisé dans l'invention, qui possède la capacité de se lier spécifiquement à la fois au facteur de coagulation IX et au facteur de coagulation X possède préférentiellement la capacité d'agir comme substitut du facteur VIII (FVIII), ce qui signifie que l'anticorps favorise l'activation du FX par le FIXa.

De tels anticorps multispécifiques, de préférence bispécifiques, peuvent être obtenus par diverses méthodes connues de l'homme du métier, par exemple par conjugaison chimique, ou en utilisant des quadromes, qui résultent de la fusion entre deux hybridomes produisant deux anticorps monoclonaux différents ; ou encore par recombinaison génétique.

Les polynucléotides codant pour de tels anticorps peuvent ainsi être insérés dans des vecteurs d'expression et exprimés dans des cellules ou organismes hôtes adaptés par des techniques bien connues de l'homme du métier.

Les anticorps utiles ici peuvent être de format très simple, construits à partir de fragments Fv à simple chaîne (scFv) de deux anticorps ou plus, associés par un linker peptidique adapté. Par « Fv », il est entendu le plus petit fragment d'anticorps conservant les propriétés de reconnaissance et de liaison à l'antigène. Un fragment « Fv » est un dimère (dimère V_{H} + V_{L}) constitué d'une région variable (V_{H}) portée par une chaîne lourde (H) et d'une région variable (V_{L}) adjacente portée par une chaîne légère (L).

Alternativement, il peut s'agir d'anticorps de pleine longueur, de préférence contenant une région Fc. Plusieurs formats sont possibles. Par exemple, dans un premier format, des fragments scFv d'un anticorps A sont fusionnés aux extrémités (en général N-terminales) des chaînes lourdes d'un anticorps B. L'anticorps résultant a un seul type de chaîne lourde, qui contient les domaines VH, CH1, CH2 et CH3 de l'anticorps B et les domaines VH et VL de l'anticorps A, et un seul type de chaîne légère qui contient les domaines VL et CL de l'anticorps B (Qu et al. Blood, 111, 2211-2219, 2008). Dans un deuxième format, la chaîne lourde et la chaîne légère d'un anticorps A sont associées à la chaîne lourde et la chaîne légère d'un anticorps B. Le cas échéant, des mutations, par exemple de type "knob into holes" (Ridgway et al, Protein Eng, 9, 617-21, 1996; brevet US 7,695,936) peuvent être introduites pour éviter les mésappariements.

Sauf indication contraire, dans la présente description, le terme « Facteur IX » se réfère indifféremment au Facteur IX non activé ou au Facteur IX activé (FIXa).

Sauf indication contraire, dans la présente description, le terme « Facteur X » se réfère indifféremment au Facteur X non activé ou au Facteur X activé (FXa).

Un anticorps reconnaissant (i) le FIX et/ou le FIXa, et (ii) le FX et/ou le FXa peut être obtenu notamment selon les méthodes décrites dans les demandes de brevets WO2005/035756, WO2006/109592 ou WO2012/067176.

Dans un mode de réalisation préféré, ledit anticorps est l'emicizumab. La production de cet anticorps est décrite par exemple dans la demande de brevet WO2018047813 ou la demande de brevet EP1688488.

### Compositions pharmaceutiques et posologies

Facteur VII et anticorps peuvent être formulés sous la forme de compositions pharmaceutiques séparées, ou combinés au sein d'une même composition pharmaceutique. Dans le cas d'administration séparée, FVII et anticorps peuvent être formulés de manière adaptée à une administration selon des voies différentes ou identiques.

Ainsi le FVII peut être administré par exemple par voie intraveineuse, sous-cutanée, ou intramusculaire.

L'anticorps peut être administré également par exemple par voie intraveineuse, sous-cutanée, ou intramusculaire.

Une composition de Facteur VII peut par exemple être telle que celle décrite dans la demande de brevet WO2010/149907.

Ainsi, dans un exemple de réalisation, la composition comprend :
- facteur VII, de préférence sous forme de facteur Vlla ;
- arginine, éventuellement sous forme de chlorhydrate ;
- isoleucine ;
- lysine ;
- glycine ;
- citrate trisodique ou chlorure de calcium ;
- et le cas échéant du polysorbate 80 ou polysorbate 20.

Plus particulièrement, la composition peut comprendre :
- facteur VII, de préférence sous forme de facteur Vlla ;
- de 10 à 40 g/l d'arginine, éventuellement sous forme de chlorhydrate ;
- de 4,2 à 6,6 g/l d'isoleucine ;
- de 0,6 à 1,8 g/l de lysine ;
- de 0,6 à 1,8 g/l de glycine ;
- de 0 à 0,2 g/l de citrate trisodique ou de 1 à 2 g/L de chlorure de calcium ;
- et le cas échéant de 0 à 0,5 g/l de polysorbate 80.

La composition de FVII, qui comprend éventuellement également au moins un anticorps multispécifique tel que décrit ici, peut être stockée sous forme liquide ou sous forme solide, obtenue typiquement par dessication. Les concentrations divulguées ci-dessus sont déterminées vis-à-vis des compositions sous forme liquide, avant dessication, ou après reconstitution sous forme de préparation injectable.

La dessiccation est un procédé d'élimination de l'eau à un stade poussé. Il s'agit d'une déshydratation visant à éliminer autant d'eau que possible. Ce phénomène peut être naturel ou forcé. Cette dessiccation peut être réalisée à l'aide des techniques de lyophilisation, d'atomisation et de cryoatomisation.

Le mode préféré d'obtention de la forme solide de la composition à usage pharmaceutique décrite ici est la lyophilisation.

Les méthodes de lyophilisation sont bien connues de l'homme du métier, voir par exemple [Wang et al, Lyophilization and development of solid protein pharmaceuticals, International Journal of Pharmaceutics, Vol 203, p 1-60, 2000].

D'autres procédés appropriés pour réduire le degré d'humidité ou la teneur en eau de la composition sont envisageables. De préférence le degré d'humidité est inférieur ou égal à 3% en poids, de préférence inférieur ou égal à 2,5%, de préférence inférieur ou égal à 2%, de préférence inférieur ou égal à 1,5%.

La composition solide, de préférence sous forme lyophilisée, peut être dissoute dans de l'eau pour préparations injectables (ou « water for injection ou WFI »), pour obtenir une formulation à usage thérapeutique.

La formulation injectable peut être administrée par voie parentérale (intraveineuse, sous-cutanée, intramusculaire), en une quantité appréciée par le praticien. L'administration de la forme liquide (avant dessiccation) ou de la forme solide, par toute voie et tout moyen appropriés, n'est pas exclue.

Le dosage de FVII utile dans l'invention, peut être déterminé de façon appropriée selon le type de formulation, la méthode d'administration, l'âge et le poids du patient, les symptômes du patient, la gravité de la maladie, etc.

La dose de FVII à administrer selon l'invention peut avantageusement être choisie entre 270 µg/kg et 2.70 µg/kg. De façon préférée la dose de FVII à administrer est inférieure à 270 µg/kg de poids corporel, de façon préférée elle est inférieure à 225 µg/kg de poids corporel, de façon préférée elle est inférieure à 180 µg/kg de poids corporel, de façon préférée elle est inférieure à 135 µg/kg de poids corporel, de façon préférée elle est inférieure à 90 µg/kg de poids corporel, de façon préférée elle est inférieure à 45 µg/kg de poids corporel, de façon préférée elle est inférieure à 9 µg/kg, de manière préférée elle est inférieure à 5.4 µg/kg, de manière préférée elle est inférieure à 2.7µg/kg.

Une composition d'anticorps multispécifique, comme l'anticorps emicizumab, est par exemple telle que celles décrites dans les demandes de brevet WO2017/188356 et WO2018/047813.

Ainsi, dans un exemple de réalisation, la composition est une composition liquide.

Dans un exemple de réalisation la composition comprend :
- anticorps bispécifique du facteur IX et du facteur X,
- un surfactant tel que le poloxamer 188 ou de polysorbate 20
- tampon histidine - acide aspartique
- arginine

Plus particulièrement, la composition peut comprendre :
- de 20 mg/mL à 180 mg/mL d'anticorps bispécifique du facteur IX et du facteur X,
- de 0.2 mg/mL à 1 mg/mL de poloxamer 188,
- de 10 mM à 40 mM de tampon histidine-acide aspartique
- de 100 mM à 300 mM d'arginine,
à un pH compris entre 4.5 et 6.5

Le dosage de la composition d'anticorps multispécifique, tel que l'anticorps emicizumab, utile dans l'invention, peut être déterminée de façon appropriée selon le type de formulation, la méthode d'administration, l'âge et le poids du patient, les symptômes du patient, la gravité de la maladie, etc. La dose d'anticorps peut être par exemple de 0.3 à 5 mg/kg, de préférence d'au maximum 3 mg/kg une fois par semaine pendant une période d'initiation, qui peut durer par exemple 4 semaines, suivie d'une dose de maintenance, qui est de préférence plus réduite, par exemple de 1.5 mg/kg une fois par semaine. De façon préférée la dose d'anticorps administrée est inférieure à 5 mg/kg de poids corporel, de façon préférée elle est inférieure à 3 mg/kg de poids corporel, de façon préférée elle est inférieure à 1.5 mg/kg de poids corporel, de façon préférée elle est inférieure à 1 mg/kg de poids corporel, de façon préférée elle est inférieure à 0.5 mg/kg de poids corporel, de façon préférée elle est inférieure à 0.1 mg/kg de poids corporel, de façon préférée elle est inférieure à 0.05 mg/kg de poids corporel.

La composition d'anticorps utile dans l'invention peut être administrée à un patient selon n'importe quelle voie appropriée, par exemple, par voie intraveineuse, intramusculaire, intrapéritonéale, intra-cérébrospinale, transdermale, sous-cutanée, intra-articulaire, sublinguale, intra-synoviale, orale ou par inhalation. De façon préférée, la voie intraveineuse ou la voie sous-cutanée est privilégiée.

Selon un mode de réalisation particulier, le facteur VII et l'anticorps sont administrés simultanément au patient.

Selon un autre mode de réalisation particulier, le facteur VII et l'anticorps sont administrés séparément chez un patient, de préférence de manière séquentielle.

### Indications thérapeutiques

La combinaison décrite ici permet de prévenir ou traiter des désordres de la coagulation, notamment des hémophilies présentant un déficit en facteur VIII (hémophilie de type A, de préférence hémophilie A acquise).

De préférence les patients sont des patients qui présentent une hémophilie de type A, avec anti-Facteur VIII.

La combinaison décrite ici permet de prévenir ou traiter des désordres de la coagulation, notamment des déficiences en facteurs VII.

La combinaison décrite ici permet d'associer l'effet rapide du FVII activant la voie extrinsèque de la cascade de la coagulation et l'effet prolongé des anticorps multispécifiques décrits ici qui activent la voie intrinsèque de la cascade de la coagulation. La combinaison permet d'offrir une meilleure prise en charge des patients.

### Exemples :

### Exemple 1 : Purification et extraction du FVII transgénique

Le procédé de purification et d'extraction du facteur VII mis en oeuvre dans cet exemple est celui décrit dans la demande EP12305882. Les étapes de ce procédé sont décrites ci-après. Le lait de lapines transgéniques est obtenu à partir de la lignée de lapines transgéniques. Le lait de lapines transgéniques congelé est décongelé et concentré sous forme de pool de lait de lapines transgéniques.

Le pool de lait de lapines transgéniques ainsi obtenu est ensuite soumis à une étape de clarification utilisant un filtre en profondeur ayant une porosité de 0,2 µm, afin d'éliminer les lipides et les composés insolubles. Le lait ainsi clarifié est ensuite soumis à une étape d'inactivation virale par traitement par un solvant de type détergent, par exemple Polysorbate 80 ou Tri-n-Butyl Phosphate à 25°C ± 2°C pendant au moins deux heures. Un tel traitement permet notamment d'inactiver efficacement les virus et en particulier, les virus de type virus non-enveloppés. Le lait clarifié et viralement inactivé est ensuite soumis à une étape de chromatographie d'affinité utilisant un ligand d'affinité spécifique du facteur VII/facteur VIIa. L'éluat de facteur VII obtenu à l'issue de cette étape de chromatographie est ensuite soumis à une étape d'ultrafiltration et de formulation, permettant ainsi d'obtenir un concentré de facteur VII intermédiaire ayant une pureté de 95%.

Le concentré de facteur VII intermédiaire est ensuite soumis à une étape de filtration utilisant un filtre ayant une porosité de 0,1 µm à 0,2 µm suivi d'une étape de nanofiltration sur des filtres ayant une porosité de 20 nm puis 15 nm. Le produit ainsi obtenu et contenant le facteur VII est ensuite soumis à une étape de chromatographie de type gel Q Sepharose XL puis à une étape de chromatographie de type CHT-I suivie d'une chromatographie de type SEC Superdex 200. Le concentré de facteur VII ainsi obtenu est ensuite soumis à une étape de stabilisation puis de filtration sur filtre ayant une porosité de 0,2 µm.

Le procédé ainsi décrit permet d'obtenir un concentré de facteur VII ayant une pureté d'environ 99,9995%.

### Exemple 2 : Comparaison du potentiel thrombogéne de Novoseven^{®}, Sevenfact^{®} et Hemlibra^{®}

L'Homme du Métier peut mesurer le potentiel thrombogéne de Novoseven^{®}, Sevenfact^{®} et Hemlibra^{®} (appelé aussi Emicizumab) en réalisant le protocole suivant.

### Réactifs :

▪ calibrateur thrombine (Stago)
▪ réactif PPP 5 pM (Stago)
▪ réactif PPP low (Stago)
▪ CK-Prest (Stago)
▪ Fluo-buffer (Stago)
▪ Fluo-substrat (Stago)
▪ Plasma déficient en FVIII (Siemens)
▪ Sevenfact^{®}/Facteur VII transgénique produit chez le lapin 1 mg/ml (LFB)
▪ PNP (Cryopep)
▪ Novoseven^{®} (NovoNordisk)
▪ Hemlibra^{®}/Emicizumab (Roche / Genentech / Chugaï)

### Méthode :

Le test de génération de thrombine consiste à activer la coagulation ex vivo soit avec un mélange de facteur tissulaire et de phospholipides (TF / PL), soit en utilisant de la céphaline puis en mesurant la concentration de thrombine générée au cours du temps.

### • Mesure du potentiel thrombogène de Novoseven^{®} après induction de la coagulation avec TF / PL :

Le test de génération de thrombine est réalisé sur 80 µL d'un pool de plasma déficient en FVIII qui mime un plasma d'hémophilie A en présence de 20 µL de réactif PPP (Stago) contenant 0,5 pM de Facteur Tissulaire (TF) et 4 µM de phospholipides (PL). La réaction est initiée par l'ajout de 20 µL de Fluca-kit (substrat + CaCl₂) qui est le début de la mesure de la génération de thrombine.

La dose thérapeutique de FVlla est de 270 µgkg, ce qui correspond à 6 µg/mL de FVlla dans le plasma, en considérant une récupération de 100%. Le test de génération de thrombine est réalisé aux doses de Novoseven^{®} de 0 µg/mL, 1 µg/mL, 2 µg/mL, 3 µg/mL, 4 µg/mL, 5 µg/mL, et 6 µg/mL, en présence de 0,5 pM TF / 2 µM PL (inducteur de la coagulation).

### • Mesure du potentiel thrombogène de Novoseven^{®} après induction de la coagulation avec la céphaline :

Le test de génération de thrombine est réalisé sur 80 µL d'un pool de plasma déficient en FVIII qui mime un plasma d'hémophilie A en présence de 20 µL de céphaline (CK-Prest reconstituée avec 5 mL de H₂0 distillée).

La réaction est initiée par l'ajout de 20 µL de Fluca-kit (substrat + CaCl₂) qui est le début de la mesure de la génération de thrombine.

Le test de génération de thrombine est réalisé aux doses de Novoseven^{®} de 0 µg/mL, 1 µg/mL, 2 µg/mL, 3 µg/mL, 4 µg/mL, 5 µg/mL, et 6 µg/mL, en présence de 20 µL de céphaline (inducteur de la coagulation).

### • Mesure du potentiel thrombogène de Sevenfact^{®} après induction de la coagulation avec TF / PL :

Le test de génération de thrombine est réalisé sur 80 µL d'un pool de plasma déficient en FVIII qui mime un plasma d'hémophilie A en présence de 20 µL de réactif PPP (Stago) contenant 0,5 pM de Facteur Tissulaire (TF) et 4 µM de phospholipides (PL).

La réaction est initiée par l'ajout de 20 µL de Fluca-kit (substrat + CaCl₂) qui est le début de la mesure de la génération de thrombine.

Le test de génération de thrombine est réalisé aux doses de Sevenfact^{®} de 0 µg/mL, 1 µg/mL, 2 µg/mL, 3 µg/mL, 4 µg/mL, 5 µg/mL, et 6 µg/mL, en présence de 0,5 pM TF / 2 µM PL (inducteur de la coagulation).

### • Mesure du potentiel thrombogène de Sevenfact^{®} après induction de la coagulation avec la céphaline :

Le test de génération de thrombine est réalisé sur 80 µL d'un pool de plasma déficient en FVIII qui mime un plasma d'hémophilie A en présence de 20 µL de céphaline (CK-Prest reconstituée avec 5 mL de H₂0 distillée).

La réaction est initiée par l'ajout de 20 µL de Fluca-kit (substrat + CaCl₂) qui est le début de la mesure de la génération de thrombine.

Le test de génération de thrombine est réalisé aux doses de Sevenfact^{®} de 0 µg/mL, 1 µg/mL, 2 µg/mL, 3 µg/mL, 4 µg/mL, 5 µg/mL, et 6 µg/mL, en présence de 20 µL de céphaline.

### • Mesure du potentiel thrombogène d'Hemlibra^{®} après induction de la coagulation avec TF / PL :

Le test de génération de thrombine est réalisé sur 80 µL d'un pool de plasma déficient en FVIII qui mime un plasma d'hémophilie A en présence de 20 µL de réactif PPP (Stago) contenant 0,5 pM de Facteur Tissulaire (TF) et 4 µM de phospholipides (PL).

La réaction est initiée par l'ajout de 20 µL de Fluca-kit (substrat + CaCl₂) qui est le début de la mesure de la génération de thrombine.

Hemlibra^{®} (Roche / Genentech / Chugaï, USA), un anticorps bispécifique mimant la fonction du FVIII, est utilisé à la concentration maximale de 50 µg/mL qui est la concentration détectée chez le patient sous traitement (Oldenburg et al. NEJM, 2017). Le test de génération de thrombine est réalisé aux doses de Hemlibra^{®} de 0 µg/mL, 10 µg/mL, 20 µg/mL, 30 µg/mL, 40 µg/mL et 50 µg/mL, en présence de 0,5 pM TF / 4 µM PL (inducteur de la coagulation).

### • Mesure du potentiel thrombogène d'Hemlibra^{®} après induction de la coagulation avec la céphaline

Le test de génération de thrombine est réalisé sur 80 µL d'un pool de plasma déficient en FVIII qui mime un plasma d'hémophilie A en présence de 20 µL de céphaline (CK-Prest reconstituée avec 5 mL de H₂0 distillée).

La réaction est initiée par l'ajout de 20 µL de Fluca-kit (substrat + CaCl₂) qui est le début de la mesure de la génération de thrombine.

Le test de génération de thrombine est réalisé aux doses de Hemlibra^{®} de 0 µg/mL, 10 µg/mL, 20 µg/mL, 30 µg/mL, 40 µg/mL et 50 µg/mL, en présence de 20 µL de céphaline.

Pour l'ensemble de ces tests, l'apparition de la fluorescence est mesurée sur un fluorimètre Fluoroskan Ascent (ThermoLabsystems) à une longueur d'onde d'excitation de 390 nm et à une longueur d'émission de 460 nm. Les thrombinogrammes (courbes représentant l'intensité de la fluorescence dans le temps) sont ensuite analysés en utilisant le logiciel Thrombinoscope ^{™}, qui convertit la valeur de fluorescence en nM de thrombine par un calcul comparatif.

La génération de thrombine est effectuée et les variables clés pour évaluer la puissance des différents médicaments sont enregistrées et comparées: potentiel de thrombine endogène (ETP), hauteur de pic, temps de latence et vélocité.

### Exemple 3: Evaluation des potentiels thrombogènes synergiques de Novoseven^{®} et Hemlibra^{®} ou SevenFact^{®} et Hemlibra^{®}

L'Homme du Métier peut mesurer le potentiel thrombogéne des combinaisons Novoseven^{®}/Hemlibra^{®} et Sevenfact^{®}/Hemlibra^{®} en réalisant le protocole suivant.

### Réactifs :

Les réactifs, l'automate et le protocole expérimental dans le plasma déficient en FVIII sont identiques à ceux décrits dans l'exemple 2.

### Méthode :

### • Mesure du potentiel thrombogène de la combinaison Novoseven^{®} + Hemlibra^{®} après induction de la coagulation avec TF / PL :

Le test de génération de thrombine est réalisé sur 80 µL d'un pool de plasma déficient en FVIII qui mime un plasma d'hémophilie A en présence de 20 µL de réactif PPP (Stago) contenant 0,5 pM de Facteur Tissulaire (TF) et 4 µM de phospholipides (PL).

La réaction est initiée par l'ajout de 20 µL de Fluca-kit (substrat + CaCl₂) qui est le début de la mesure de la génération de thrombine.

Le test de génération de thrombine est réalisé en présence de 0,5 pM TF / 4 µM PL (inducteur de la coagulation) sur plusieurs combinaisons Novoseven^{®}/Hemlibra^{®}. La composition contenant la plus grande quantité de produit est constituée de 6 µg/mL de Novoseven^{®} et de 50 µg/mL d'Hemlibra^{®}, à leur maximum.

Le potentiel thrombogène obtenu en présence de la combinaison de produits est comparé au potentiel des produits uniques. Pour envisager un effet synergique de la combinaison de produit, des doses plus faibles sont évaluées pour être sûr de ne pas saturer la détection de la thrombine.

Les compositions testées contiennent :

| Combinaison | Hemlibra^{®} (µg/mL) | NovoSeven^{®} (µg/mL) |
|---|---|---|
| Combinaison 1 | 50 | 6 |
| Combinaison 2 | 50 | 5 |
| Combinaison 3 | 40 | 4 |
| Combinaison 4 | 30 | 3 |
| Combinaison 5 | 20 | 2 |
| Combinaison 6 | 10 | 1 |

### • Mesure du potentiel thrombogène de la combinaison Novoseven^{®} + Hemlibra^{®} après induction de la coagulation avec la céphaline

Le test de génération de thrombine est réalisé sur 80 µL d'un pool de plasma déficient en FVIII qui mime un plasma d'hémophilie A en présence de 20 µL de céphaline (CK-Prest reconstituée avec 5 mL de H₂0 distillée).

La réaction est initiée par l'ajout de 20 µL de Fluca-kit (substrat + CaCl₂) qui est le début de la mesure de la génération de thrombine.

Le test de génération de thrombine est réalisé en présence de 20 µL de céphaline sur plusieurs combinaisons Novoseven^{®}/Hemlibra^{®}. La composition contenant la plus grande quantité de produit est constituée de 6 µg/mL de Novoseven^{®} et de 50 µg/mL d'Hemlibra^{®}, à leur maximum.

Le potentiel thrombogène obtenu en présence de la combinaison de produits est comparé au potentiel des produits uniques. Pour envisager un effet synergique de la combinaison de produit, des doses plus faibles sont évaluées pour être sûr de ne pas saturer la détection de la thrombine.

Les compositions testées contiennent :

| Combinaison | Hemlibra^{®} (µg/mL) | NovoSeven^{®} (µg/mL) |
|---|---|---|
| Combinaison 1 | 50 | 6 |
| Combinaison 2 | 50 | 5 |
| Combinaison 3 | 40 | 4 |
| Combinaison 4 | 30 | 3 |
| Combinaison 5 | 20 | 2 |
| Combinaison 6 | 10 | 1 |

### • Mesure du potentiel thrombogène de la combinaison de Sevenfact^{®} + Hemlibra^{®} après induction de la coagulation avec TF / PL

Le test de génération de thrombine est réalisé sur 80 µL d'un pool de plasma déficient en FVIII qui mime un plasma d'hémophilie A en présence de 20 µL de réactif PPP (Stago) contenant 0,5 pM de Facteur Tissulaire (TF) et 4 µM de phospholipides (PL).

La réaction est initiée par l'ajout de 20 µL de Fluca-kit (substrat + CaCl₂) qui est le début de la mesure de la génération de thrombine.

Le test de génération de thrombine est réalisé en présence de 0,5 pM TF / 4 µM PL (inducteur de la coagulation) sur plusieurs combinaisons Sevenfact^{®}/Hemlibra^{®}. La composition contenant la plus grande quantité de produit est constituée de 6 µg/mL de Sevenfact^{®} et de 50 µg/mL d'Hemlibra^{®}, à leur maximum.

Le potentiel thrombogène obtenu en présence de la combinaison de produits est comparé au potentiel des produits uniques. Pour envisager un effet synergique de la combinaison de produit, des doses plus faibles sont évaluées pour être sûr de ne pas saturer la détection de la thrombine.

Les compositions testées contiennent :

| Combinaison | Hemlibra^{®} (µg/mL) | **Sevenfact^{®}** (µg/mL) |
|---|---|---|
| Combinaison 1 | 50 | 6 |
| Combinaison 2 | 50 | 5 |
| Combinaison 3 | 40 | 4 |
| Combinaison 4 | 30 | 3 |
| Combinaison 4 | 20 | 2 |
| Combinaison 6 | 10 | 1 |

### • Mesure du potentiel thrombogène de la combinaison Sevenfact^{®} + Hemlibra^{®} après induction de la coagulation avec la céphaline

Le test de génération de thrombine est réalisé sur 80 µL d'un pool de plasma déficient en FVIII qui mime un plasma d'hémophilie A en présence de 20 µL de céphaline (CK-Prest reconstituée avec 5 mL de H₂0 distillée).

La réaction est initiée par l'ajout de 20 µL de Fluca-kit (substrat + CaCl₂) qui est le début de la mesure de la génération de thrombine.

Le test de génération de thrombine est réalisé en présence de 20 µL de céphaline sur plusieurs combinaisons Sevenfact^{®}/Hemlibra^{®}. La composition contenant la plus grande quantité de produit est constituée de 6 µg/mL de Sevenfact^{®} et de 50 µg/mL d'Hemlibra^{®} à leur maximum.

Le potentiel thrombogène obtenu en présence de la combinaison de produits est comparé au potentiel des produits uniques. Pour envisager un effet synergique de la combinaison de produit, des doses plus faibles sont évaluées pour être sûr de ne pas saturer la détection de la thrombine.

Les compositions testées contiennent :

| Combinaison | Hemlibra^{®} (µg/mL) | **Sevenfact^{®}** (µg/mL) |
|---|---|---|
| Combinaison 1 | 50 | 6 |
| Combinaison 2 | 50 | 5 |
| Combinaison 3 | 40 | 4 |
| Combinaison 4 | 30 | 3 |
| Combinaison 5 | 20 | 2 |
| Combinaison 6 | 10 | 1 |

Pour l'ensemble de ces tests, l'apparition de la fluorescence est mesurée sur un fluorimètre Fluoroskan Ascent (ThermoLabsystems) à une longueur d'onde d'excitation de 390 nm et à une longueur d'émission de 460 nm. Les thrombinogrammes (courbes représentant l'intensité de la fluorescence dans le temps) sont ensuite analysés en utilisant le logiciel Thrombinoscope ^{™}, qui convertit la valeur de fluorescence en nM de thrombine par un calcul comparatif.

Un effet synergique est par exemple considéré lorsqu'au moins l'un des paramètres calculés à partir du test de génération de thrombine pour une combinaison donnée est supérieur à la somme de chacun de ces paramètres obtenus avec les composants seuls, déduits du bruit de fond de l'expérience.

### Exemple 4 : Comparaison du potentiel de Sevenfact^{™}, d'Hemlibra^{®} et de la combinaison des deux dans du plasma hémophile A:

### Réactifs :

▪ calibrateur thrombine (Stago)
▪ réactif PRP reagent 1pM TF (Stago)
▪ réactif MP 4µM PL (Stago)
▪ Fluo-buffer (Stago)
▪ Fluo-substrate (Stago)
▪ Sevenfact^{™} : Facteur VII transgénique produit chez le lapin 1 mg/mL (LFB)
▪ Hemlibra^{®}: Emicizumab (Roche / Genentech / Chugaï)
▪ Plasma Hémophile A (Cryopep)
▪ Owren Koller (Stago)

### Méthode :

Le test de génération de thrombine consiste à activer la coagulation *ex vivo,* par exemple avec un mélange de facteur tissulaire et de phospholipides (TF / PL), puis à mesurer la concentration de thrombine générée au cours du temps. Les tests de génération de thrombine sont réalisés avec 80µL de plasma hémophile A (Cryopep), en présence de 20 µL d'un mélange des réactifs PRP et MP (Stago) contenant 0.5 pM de facteur tissulaire et 4 µM de phospholipides.

La réaction est initiée par addition de 20 µL de Fluca-kit (Fluo substrate + CaCl₂), ce qui correspond au point de départ de la mesure de la génération de thrombine (TG).

La fluorescence est mesurée par fluorimétrie à l'aide de l'appareil Fluoroskan Ascent (ThermoLabsystems) à une longueur d'onde d'excitation de 390 nm et une longueur d'onde d'émission de 460 nm. Les thrombinogrammes sont analysés en utilisant le logiciel Thrombinoscope^{™} qui convertit par calcul comparatif l'intensité de fluorescence en concentration molaire de thrombine (nM).

Pour mesurer le potentiel thrombogène des deux molécules, plusieurs plasmas Hémophile A sont étudiés. La dose thérapeutique la plus élevée de FVlla est de 270 µg/kg, ce qui correspond à 6 µg/mL de FVlla (ou 120 nM) dans le plasma. L'utilisation de cette dose peut être considérée comme un potentiel maximal de génération de thrombine. Sur la base des concentrations de produit en circulation obtenues chez les patients, des concentrations de Sevenfact^{™} comprises entre 20 et 100 nM sont aussi étudiées. Hemlibra^{®} (Roche / Genentech / Chugaï, USA), un anticorps bispécifique imitant la fonction du FVIII, est utilisé à la concentration maximale de 120 µg/mL. La concentration actuellement détectée chez les patients sous traitement est de 50 µg/mL (ou 300 nM) (Oldenburg et al. NEJM, 2017). Ainsi, Hemlibra^{®} est utilisé ici à environ 300 nM (50 µg/mL). Les variables étudiées pour mesurer le potentiel thrombogène de Hemlibra^{®}, de Sevenfact^{™} sont :
- le potentiel de thrombine endogène (PTE) : aire sous la courbe représentant la quantité totale de thrombine générée,
- la hauteur de pic : concentration maximale de thrombine mesurée, et
- la vélocité de génération de thrombine : vitesse de formation de thrombine.

### 2 - Résultats

### 2.1 - Effet de Sevenfact^{™} ou Hemlibra^{®} sur des plasmas Hémophile A

### 2.1.1 - Évaluation dans le lot 1 de plasma Hémophile A

Dans cette matrice, de très faibles signaux de génération de thrombine provenant des deux composés sont obtenus, quelles que soient les concentrations utilisées. En effet, la génération de thrombine observée est quasiment nulle pour Hemlibra^{®} et Sevenfact^{™} aux concentrations de 20 et 40 nM. Avec 100 nM de Sevenfact^{™}, un pic de génération de thrombine très faible est observé (Tableau 1).

**Tableau 1 : Paramètres de génération de thrombine à partir du lot 1 de plasma Hémophile A traité avec Sevenfact^{™} ou Hemlibra^{®}**

| | Sevenfact^{™} 20nM | Sevenfact^{™} 40nM | Sevenfact^{™} 100nM | Hemlibra^{®} 300nM |
|---|---|---|---|---|
| PTE (nM.min) | 125 | 154.25 | 128.75 | 128.25 |
| Pic (nM) | 7.105 | 9.46 | 16.55 | 6.41 |
| Vélocité (nM/min) | 0.59 | 0.84 | 1.64 | 0.42 |

Ainsi, chaque molécule utilisée individuellement n'induit qu'une très faible génération de thrombine.

### 2.1.2 - Évaluation dans le lot 2 de plasma Hémophile A

Un second lot de plasma Hémophile A a été testé. Là encore, une très faible génération de thrombine est observée avec l'utilisation de Hemlibra^{®} et Sevenfact^{™}, avec un pic de génération de thrombine maximum à une concentration de 100 nM de Sevenfact^{™} (Tableau 2).

**Tableau 2 : Paramètres de génération de thrombine à partir du lot 2 de plasma Hémophile A traité avec Sevenfact^{™} ou Hemlibra^{®}**

| | Sevenfact^{™} 20 nM | Sevenfact^{™} 40 nM | Sevenfact^{™} 100 nM | Hemlibra^{®} 300 nM |
|---|---|---|---|---|
| PTE (nM.min) | 221.25 | 280.75 | 414.25 | 196 |
| Pic (nM) | 9.93 | 12.84 | 21.04 | 8.895 |
| Vélocité (nM/min) | 0.7 | 0.96 | 1.75 | 0.51 |

Dans cette matrice, Sevenfact^{™} et Hemlibra^{®} utilisés séparément ont un faible potentiel thrombogène.

### Exemple 5 : Evaluation du potentiel thrombogène synergique de la combinaison Sevenfact^{™} + Hemlibra^{®}

### 1 - Protocole

Les réactifs, l'automate et le protocole expérimental dans le plasma de l'hémophilie A sont identiques à ceux décrits dans l'exemple 2.

### 2 - Résultats

Comme vu dans l'exemple 2, Sevenfact^{™} et Hemlibra^{®} utilisés individuellement induisent une faible génération de thrombine dans les plasmas Hémophile A. L'effet synergique de la combinaison Sevenfact^{™} et Hemlibra^{®} est étudié ici. Trois concentrations de Sevenfact^{™} sont étudiées (20 nM, 40 nM et 100 nM) en présence d'une concentration de Hemlibra^{®} de 300 nM. Un effet synergique est pris en compte si l'effet de la combinaison Sevenfact^{™} + Hemlibra^{®} est au moins 2 fois supérieur à la somme des effets de Sevenfact^{™} et de Hemlibra^{®} pris séparément pour au moins un des paramètres du test de génération de thrombine (PTE, pic de génération de thrombine, et vélocité).

### 2.1 - Effet de Sevenfact^{™} et d'Hemlibra^{®} sur les plasmas Hémophile A après induction de la coagulation avec TF / PL

### 2.1.1 - Évaluation dans le lot 1 de plasma de l'hémophilie A

Les résultats sont présentés dans le tableau 3 et la figure 1. A une concentration très faible de Sevenfact^{™} de 20 nM, les ratios pour le PTE (figure 1A), pour le pic de thrombine (figure 1B) et pour la vélocité (figure 1C) de la combinaison Sevenfact^{™} + Hemlibra^{®} sont respectivement de 2.14, 2.95 et 4.19. Ainsi, même à la plus faible concentration testée, un effet thrombogène synergique est observé.

A une concentration de 40 nM, pour l'ensemble des paramètres testés le ratio est supérieur à 2. Le ratio obtenu pour le PTE est de 2.75 (figure 1A), le ratio obtenu pour le pic de thrombine est de 3.96 (figure 1B) et celui obtenu pour la vélocité atteint une valeur de 6.21 (figure 1C). Autrement dit, la vitesse de formation de la thrombine est multipliée par 6 lorsque Sevenfact^{™} et Hemlibra^{®} sont utilisés en combinaison.

C'est à une concentration de 100 nM de Sevenfact^{™} que l'effet synergique est le plus important. A une concentration de 100 nM, pour l'ensemble des paramètres testés le ratio est supérieur à 2. Le ratio obtenu pour le PTE est de 4.00 (figure 1A) et le ratio pour le pic de thrombine est de 4.81 (figure 1B), ce qui signifie que la concentration maximale de thrombine générée est presque 5 fois plus importante lorsque Hemlibra^{®} et Sevenfact^{™} sont utilisés en combinaison. Le ratio correspondant à la vélocité est de 9.58 (figure 1C), ce qui signifie que la thrombine est générée presque 10 fois plus rapidement lorsque Sevenfact^{™} et Hemlibra^{®} sont utilisés en combinaison.

**Tableau 3: Paramètres de génération de thrombine à partir du lot 1 de plasma Hémophile A traité avec la combinaison Sevenfact^{™} + Hemlibra^{®}**

| | | | Combinaisons | | | Ratios combinaisons/somme | | |
|---|---|---|---|---|---|---|---|---|
| PTE 20nM Sevenfact^{™} + PTE 300nM Hemlibra^{®} | PTE 40nM Sevenfact^{™} + PTE 300nM Hemlibra^{®} | PTE 100nM Sevenfact^{™} + PTE 300nM Hemlibra^{®} | PTE (20nM Sevenfact^{™} + 300nM Hemlibra^{®}) | PTE (40nM Sevanfact^{™} + 300nM Hemlibra^{®}) | PTE (100nM Sevenfact^{™} + 300nM Hemlibra^{®}) | 20nM Sevenfact^{™} | 40nM Sevenfact^{™} | 100nM Sevenfact ^{™} |
| 253.25 | 282.50 | 257 | 543 | 777.50 | 1029 | 2.14 | 2.75 | 4.00 |
| | | | | | | | | |

| | | | Combinaisons | | | Ratios combinaisons/somme | | |
|---|---|---|---|---|---|---|---|---|
| Pic 20nM Sevenfact^{™} + Pic 300nM Hemlibra^{®} | Pic 40nM Sevenfact^{™} + Pic 300nM Hemlibra^{®} | Pic 100nM Sevenfact^{™} + Pic 300nM Hemlibra^{®} | Pic (20nM Sevenfact^{™} + 300nM Hemlibra^{®}) | Pic (40nM Sevenfact^{™} + 300nM Hemlibra^{®}) | Pic (100nM Sevenfact^{™} + 300nM Hemlibra^{®}) | 20nM Sevenfact^{™} | 40nM Sevenfact^{™} | 100nM Sevenfact ^{™} |
| 13.515 | 15.87 | 22.965 | 39.915 | 62.9 | 110.565 | 2.95 | 3.96 | 4.81 |
| | | | | | | | | |

| | | | Combinaisons | | | Ratios combinaisons/somme | | |
|---|---|---|---|---|---|---|---|---|
| Vélocité 20nM Sevenfact^{™} + Vélocité 300nM Hemlibra^{®} | Vélocité 40nM Sevenfact^{™} + Vélocité 300nM Hemlibra^{®} | Vélocité 100nM Sevenfact^{™} + Vélocité 300nM Hemlibra^{®}) | Vélocité (20nM Sevenfact^{™} + 300nM Hemlibra^{®}) | Vélocité (40nM Sevenfact^{™} + 300nM Hemlibra^{®}) | Vélocité (100nM Sevenfact^{™} + 300nM Hemlibra^{®}) | 20nM Sevenfact^{™} | 40nM Sevenfact^{™} | 100nM Sevenfact ^{™} |
| 1.01 | 1.42 | 2.06 | 4.24 | 7.86 | 19.78 | 4.19 | 6.21 | 9.58 |

En conclusion, pour toutes les concentrations de Sevenfact^{™} testées, Sevenfact^{™} et Hemlibra^{®} utilisés en combinaison ont un effet synergique sur la génération de thrombine.

### 2.1.2 - Évaluation dans le lot 2 de plasma Hémophile A

Les résultats sont présentés dans le tableau 4 et la figure 2. A une concentration très faible de Sevenfact^{™} de 20 nM, un ratio de 2.21 est obtenu pour le paramètre de PTE (figure 2A), un ratio de 2.34 est obtenu pour le pic de thrombine (figure 2B), et un ratio de2.9 est obtenu pour le paramètre de vélocité (figure 2C) de la combinaison Sevenfact^{™} + Hemlibra^{®}. Ainsi, même à la plus faible concentration testée de Sevenfact^{™}, un effet thrombogène synergique est observé.

A une concentration de 40 nM, le ratio correspondant au PTE est de 2.29 (figure 2A), celui correspondant au pic de thrombine est de 2.79 (figure 2B) et le ratio correspondant à la vélocité est de 3.68 (Figure 2C), ce qui signifie que l'utilisation de Sevenfact^{™} en combinaison avec Hemlibra^{®} permet la formation de thrombine environ 4 fois plus rapidement.

C'est avec une concentration de 100 nM de Sevenfact^{™} que l'effet synergique est le plus important. A une concentration de 100 nM, le ratio correspondant au pic de génération de thrombine est de 3.41 (Figure 2B) et celui correspondant à la vélocité est de 5.63 (Figure 2C), ce qui signifie que la thrombine est générée presque 6 fois plus rapidement et que la concentration de thrombine atteinte est multipliée par presque 4 lorsque Sevenfact^{™} est utilisé en combinaison avec Hemlibra^{®}.

**Tableau 4: Paramètres de génération de thrombine à partir du lot 2 de plasma Hémophile traité avec la combinaison Sevenfact^{™} + Hemlibra^{®}**

| | | | Combinaisons | | | Ratios combinaisons/somme | | |
|---|---|---|---|---|---|---|---|---|
| PTE 20nM Sevenfact^{™} + PTE 300nM Hemlibra^{®} | PTE 40nM Sevenfact^{™} + PTE 300nM Hemlibra^{®} | PTE 100nM Sevenfact^{™} + PTE 300nM Hemlibra^{®} | PTE (20nM Sevenfact^{™} + 300nM Hemlibra^{®}) | PTE (40nM Sevenfact^{™} + 300nM Hemlibra^{®}) | PTE (100nM Sevenfact^{™} + 300nM Hemlibra^{®}) | 20nM Sevenfact^{™} | 40nM Sevenfact ^{™} | 100nM Sevenfact^{™} |
| 417.25 | 476.75 | 610.25 | 920.75 | 1091.25 | 1275.50 | 2.21 | 2.29 | 2.09 |
| | | | | | | | | |

| | | | Combinaisons | | | Ratios combinaisons/somme | | |
|---|---|---|---|---|---|---|---|---|
| Pic 20nM Sevenfact^{™} + Pic 300nM Hemlibra^{®} | Pic 40nM Sevenfact^{™} + Pic 300nM Hemlibra^{®} | Pic 100nM Sevenfact^{™} + Pic 300nM Hemlibra^{®} | Pic (20nM Sevenfact^{™} + 300nM Hemlibra^{®}) | Pic (40nM Sevenfact^{™} + 300nM Hemlibra^{®}) | Pic (100nM Sevenfact^{™} + 300nM Hemlibra^{®}) | 20nM Sevenfact^{™} | 40nM Sevenfact^{™} | 100nM Sevenfact^{™} |
| 18.825 | 21.735 | 29.935 | 43.96 | 60.745 | 101.975 | 2.34 | 2.79 | 3.41 |
| | | | | | | | | |

| | | | Combinaisons | | | Ratios combinaisons/somme | | |
|---|---|---|---|---|---|---|---|---|
| Vélocité 20nM Sevenfact^{™} + Vélocité 300nM Hemlibra^{®} | Vélocité 40nM Sevenfact^{™} + Vélocité 300nM Hemlibra^{®} | Vélocité 100nM Sevenfact^{™} + PTE 300nM Hemlibra^{®} | Vélocité (20nM Sevenfact^{™} + 300nM Hemlibra^{®}) | Vélocité (40nM Sevenfact^{™} + 300nM Hemlibra^{®}) | Vélocité (100nM Sevenfact^{™} + 300nM Hemlibra^{®}) | 20nM Sevenfact^{™} | 40nM Sevenfact^{™} | 100nM Sevenfact^{™} |
| 1.21 | 1.48 | 2.27 | 3.52 | 5.44 | 12.77 | 2.9 | 3.68 | 5.63 |

En conclusion, pour toutes les concentrations de Sevenfact^{™} testées, Sevenfact^{™} et Hemlibra^{®} utilisés en combinaison ont un effet synergique sur la génération de thrombine.

## Revendications

1. Composition pharmaceutique comprenant :
a. du Facteur VII transgénique, et
b. un anticorps multispécifique dirigé contre le facteur IX et le facteur X ;
ledit facteur VII transgénique étant un facteur VII humain issu d'une production par les cellules épithéliales des glandes mammaires d'un lapin transgénique.

2. Composition pharmaceutique selon la revendication 1, dans laquelle l'anticorps est l'emicizumab.

3. Produit de combinaison comprenant :
a. du Facteur VII transgénique, et
b. un anticorps multispécifique dirigé contre le facteur IX et le facteur X,
pour son utilisation dans la prévention ou le traitement d'un désordre de la coagulation chez un patient ;
ledit facteur VII transgénique étant un facteur VII humain issu d'une production par les cellules épithéliales des glandes mammaires d'un lapin transgénique.

4. Produit de combinaison pour son utilisation selon la revendication 3, dans le traitement de l'hémophilie A.

5. Produit de combinaison pour son utilisation selon l'une des revendications 3 ou 4, dans le traitement de l'hémophilie A avec inhibiteurs de facteur VIII.

6. Produit de combinaison pour son utilisation selon les revendications 3 à 5, ledit produit de combinaison étant sous la forme d'une composition pharmaceutique telle que définie à l'une quelconque des revendications 1 ou 2.

7. Produit de combinaison pour son utilisation selon les revendications 3 à 6, ledit facteur VIIa et ledit anticorps étant sous une forme adaptée à une administration simultanée au patient.

8. Produit de combinaison pour son utilisation selon les revendications 3 à 5, ledit facteur VIIa et ledit anticorps étant sous des formes adaptées à une administration de façon séparée audit patient.

9. Kit comprenant
- Un récipient contenant un facteur FVII transgénique, ledit facteur VII transgénique étant un facteur VII humain issu d'une production par les cellules épithéliales des glandes mammaires d'un lapin transgénique ; et
- Un autre récipient contenant un anticorps dirigé contre le facteur IX et le facteur X.

## Patentansprüche

1. Pharmazeutische Zusammensetzung umfassend:
a. transgenen Faktor VII, und
b. einen multispezifischen Antikörper, gerichtet gegen Faktor IX und Faktor X;
wobei der transgene Faktor VII ein humaner Faktor VII ist, der aus einer Produktion durch Milchdrüsenepithelzellen eines transgenen Kaninchens stammt.

2. Pharmazeutische Zusammensetzung gemäß Anspruch 1, wobei der Antikörper Emicizumab ist.

3. Kombinationsprodukt umfassend:
a. transgenen Faktor VII, und
b. einen multispezifischen Antikörper, gerichtet gegen Faktor IX und Faktor X, zur Verwendung in der Prävention oder Behandlung einer Gerinnungsstörung bei einem Patienten;
wobei dieser transgene Faktor VII ein humaner Faktor VII ist, der aus einer Produktion durch Milchdrüsenepithelzellen eines transgenen Kaninchens stammt.

4. Kombinationsprodukt zur Verwendung gemäß Anspruch 3 in der Behandlung der Hämophilie A.

5. Kombinationsprodukt zur Verwendung gemäß einem der Ansprüche 3 oder 4 in der Behandlung der Hämophilie A mit Faktor VIII-Hemmern.

6. Kombinationsprodukt zur Verwendung gemäß den Ansprüchen 3 bis 5, wobei das Kombinationsprodukt in Form einer pharmazeutischen Zusammensetzung, definiert wie in einem der Ansprüche 1 oder 2, vorliegt.

7. Kombinationsprodukt zur Verwendung gemäß den Ansprüchen 3 bis 6, wobei der Faktor VIIa und der Antikörper in einer Form vorliegen, die für eine gleichzeitige Verabreichung an den Patienten geeignet ist.

8. Kombinationsprodukt zur Verwendung gemäß den Ansprüchen 3 bis 5, wobei der Faktor VIIa und der Antikörper in Formen vorliegen, die für eine getrennte Verabreichung an den Patienten geeignet sind.

9. Kit umfassend
- einen Behälter enthaltend einen transgenen Faktor FVII, wobei dieser transgene Faktor VII ein humaner Faktor VII ist, der aus einer Produktion durch Milchdrüsenepithelzellen eines transgenen Kaninchens stammt, und
- einen weiteren Behälter enthaltend einen Antikörper, gerichtet gegen Faktor IX und Faktor X.

## Claims

1. Pharmaceutical composition comprising:
a. transgenic factor VII, and
b. a multispecific antibody directed against factor IX and factor X;
said transgenic factor VII being a human factor VII derived from production by epithelial cells of the mammary glands of a transgenic rabbit.

2. Pharmaceutical composition according to claim 1, wherein the antibody is emicizumab.

3. Combination product comprising:
a. transgenic factor VII, and
b. a multispecific antibody directed against factor IX and factor X,
for its use in preventing or treating a coagulation disorder in a patient;
said transgenic factor VII being a human factor VII derived from production by epithelial cells of the mammary glands of a transgenic rabbit.

4. Combination product for its use according to claim 3, in the treatment of hemophilia A.

5. Combination product for its use according to one of claims 3 or 4, in the treatment of hemophilia A with factor VIII inhibitors.

6. Combination product for its use according to claims 3 to 5, said combination product being in the form of a pharmaceutical composition such as defined in any one of claims 1 or 2.

7. Combination product for its use according to claims 3 to 6, said factor Vila and said antibody being in a form suited to simultaneous administration to the patient.

8. Combination product for its use according to claims 3 to 5, said factor Vila and said antibody being in forms suited to separate administration to the patient.

9. Kit comprising
- A container containing transgenic factor VII, said transgenic factor VII being a human factor VII derived from production by epithelial cells of the mammary glands of a transgenic rabbit; and
- Another container containing an antibody directed against factor IX and factor X.
